# EUROPEAN PATENT SPECIFICATION

(11) **EP 4 032 573 B1**
(45) Date of publication and mention of the grant of the patent: **08.07.2026**
(21) Application number: 20864573.9
(22) Date of filing: 02.09.2020
(51) Int. Cl.: A61M 11/00

(54) **ATOMIZER**
ZERSTÄUBER
ATOMISEUR

(30) Priority: 19.09.2019 CN 201921562175 U
(43) Date of publication of application: 27.07.2022
(73) Proprietor: Suzhou Skywell Healthcare Information Co., Ltd., Suzhou, Jiangsu 215123 (CN)
(72) Inventor: GU, Yu, Suzhou, Jiangsu 215123 (CN)
(74) Representative: Simmons & Simmons LLP (Munich)
(86) International application number: PCT/CN2020/112946
(87) International publication number: WO 2021/052173

(56) References cited:
- EP-A1- 3 501 582
- CN-A- 101 300 041
- CN-A- 105 149 135
- CN-U- 206 473 655
- CN-U- 208 307 411
- CN-U- 210 785 807
- KR-A- 20190 050 317
- US-A1- 2007 282 276
- US-A1- 2007 282 276

## Description

### TECHNICAL FIELD

The present application generally relates to fluid delivery, and more particularly, to an atomizer.

### BACKGROUND

An atomizer can be used to treat respiratory diseases by dispersing liquid medicines into tiny droplets or particles, so that patients can directly inhale the medicines into the respiratory tract, thereby acting on the surface of the respiratory tract. The jet atomizer is a common atomizer, which uses a plunger to press the liquid medicine in the pump cavity to form a high-speed air flow through the small nozzle, so as to form droplets and spray them out of an air outlet pipe. A known atomizer is disclosed in US 2007/282276 A1, which discloses a process and a device for dosing a liquid pharmaceutical agent, wherein, in order to achieve an enhanced dosing accuracy, a shaped seal is produced in batches, is combined with a guide pipe selected from a suitable group of guide pipes, and wherein the suitable group of guide pipes is selected based on at least one decisively significant value of the respective batch of shaped seals and is distinguished by an essential value of the guide pipe which will optimize the sealing between these components.

Document KR 2019 0050317 A discloses a seal used in a processing system.

However, the sealing between the plunger and the pump body of the atomizer in the prior art is poor.

### SUMMARY

An objective of the present application is to provide an atomizer having the features of claim 1, for improving the sealing between the plunger and the pump of the liquid pump of the atomizer. According to claim 1, an atomizer is provided. The atomizer of claim 1 includes: a pump housing including an internal chamber extending in an axial direction of the bump housing and a mounting seat disposed at the first end of the pump housing and being in communication with the internal chamber; a seal disposed in the mounting seat, the seal comprising a sealing sleeve and a first bore in the sealing sleeve and coaxial with the internal chamber, and at least a part of the seal resting against a mounting surface of the mounting seat; a pressing member at least partially disposed within the mounting seat and resting against the seal to press and keep the seal in the mounting seat, the pressing member including a second bore coaxial with the internal chamber; and a plunger, a first end of the plunger being disposed within the internal chamber of the pump housing through the second bore of the pressing member and the first bore of the seal, and the first end of the plunger being capable of reciprocating in the internal chamber to pump fluid in or out of the internal chamber; wherein the pump housing, the pressing member and the plunger together define a sealing cavity to accommodate the seal, and when the seal is in the sealing cavity, a fill ratio of the seal to the sealing cavity is less than 90%. In addition, the seal includes a flange protruding outward from the sealing sleeve, and the flange rests against the mounting surface of the mounting seat, and a portion of the sealing cavity away from the flange of the seal in a direction of movement of the plunger includes a gap that is not filled by the seal.

In some embodiments, a height of the gap is 5% to 60% of a height of the sealing cavity in the direction of movement of the plunger.

In some embodiments, a lug is disposed on the flange of the seal, that the lug extends in an axial direction of the sealing sleeve, the sealing cavity includes a groove that matches the lug to accommodate the lug, and a movement of the seal relative to the sealing cavity is further limited by the lug and the groove.

In some embodiments, a side wall of the first bore of the seal includes a plurality of annular corrugated protrusions surrounding the plunger.

In some embodiments, a chamfer is formed at an end of a side wall of the first bore of the seal that is away from the mounting surface.

In some embodiments, the seal includes at least one material selected from the group consisting of: rubber, silicone, or thermoplastic elastomer.

In some embodiments, the atomizer further includes an external retainer, wherein the external retainer comprises an internal thread at its an inner wall, the mounting seat of the pump housing comprises an external thread at its outer wall, and the external retainer is configured to fix the pressing member in the mounting seat through a thread connection between the internal thread and the external thread.

In some embodiments, the fill ratio of the seal to the sealing cavity is 60% to 89%.

In some embodiments, the fill ratio of the seal to the sealing cavity is 75% to 89%.

In some embodiments, the fill ratio of the seal to the sealing cavity is 80% to 89%.

In some embodiments, the atomizer further includes a reservoir, wherein the plunger comprises a fluid channel, and the fluid in the reservoir is capable of flowing through the fluid channel into the internal chamber of the pump housing.

In some embodiments, a check valve is disposed on the first end of the plunger, the check valve is configured that, when the plunger moves in a direction away from the internal chamber, the check valve is open and the fluid in the reservoir flows into the internal chamber through the fluid channel and the check valve, and when the plunger moves in a direction toward the internal chamber, the check valve is closed to block the fluid from flowing back to the fluid channel.

In some embodiments, the atomizer further includes a nozzle disposed at a second end of the pump housing opposite to the first end, the nozzle includes one or more micro-channels in communication with the internal chamber; wherein when the plunger moves in the direction toward the internal chamber, the fluid in the internal chamber is squeezed out of the atomizer through the micro-channels of the nozzle to form a jet or aerosol.

The above is an overview of the application, and there may be simplifications, generalizations and omissions of details. Therefore, a person skilled in the art may understand that the summary is explanatory only and is not restrictive of the invention. The summary is not intended to identify key features or essential features of the claimed subject matter, nor is it intended to be used as an aid in determining the scope of the claimed subject matter.

### BRIEF DESCRIPTION OF THE DRAWINGS

The above and other features of the present disclosure will be more fully understood by those skilled in the art from the following detailed description taken in conjunction with the accompanying drawings and the appended claims. It is to be understood that these drawings and detailed descriptions depict only several exemplary embodiments of the present disclosure and should not be considered as limiting the scope of the appended claims. The contents of the present application will be explained more clearly and in detail by referring to the accompanying drawings.
Fig. 1 shows a schematic diagram of an atomizer according to an embodiment of the present application;
Fig. 2 is a partial enlarged schematic diagram of the atomizer of Fig. 1;
Fig. 3 is a schematic diagram of a seal of the atomizer of Fig. 1;
Fig. 4 is a schematic diagram of a seal according to another embodiment of the present application;
Fig. 5 is a schematic diagram of an atomizer equipped with the seal of Fig. 4;
Fig. 6 is a schematic diagram of a seal according to another embodiment of the present application;
Fig. 7 is a schematic diagram of a seal according to another embodiment of the present application;
Fig. 8 is a schematic diagram of a seal according to another embodiment of the present application;
Fig. 9 is a schematic diagram of an atomizer equipped with the seal of Fig. 6;
Fig. 10 is a schematic diagram of an atomizer equipped with the seal of Fig. 7; and
Fig. 11 is a schematic diagram of an atomizer equipped with the seal of Fig. 8.

### DETAILED DESCRIPTION

In the following detailed description, reference is made to the accompanying drawings which form a part of this specification. In the drawings, similar symbols typically identify similar components, unless context dictates otherwise. The illustrative embodiments described in the detailed description and drawings are not intended to be limiting the scope of the appended claims. Other embodiments may be utilized, and other changes may be made, without departing from the scope of the appended claims. It will be appreciated that various configurations, substitutions, combinations, designs of various configurations may be made to the various aspects of the content of the application generally described in the application and illustrated in the accompanying drawings, all of which expressly constitute the subject matter of the application part.

The inventors of the present application found that the sealing between the plunger and the pump body of the atomizer in the prior art is poor, especially after a long time or repeated use, the particle size of the liquid particles produced by the atomizer are often unstable, and it is difficult to satisfy the need of effective drug delivery.

In view of the above-mentioned technical problems existing in the prior art, the inventors of the present application have conducted a large number of design analysis and experimental verification. After research, the inventors of the present application found that in order to improve the sealing between the plunger and the pump body, the atomizer in the prior art includes a seal between the pump body and the plunger, and the seal is disposed in a sealing cavity in the pump having a shape substantially matching the seal. In addition, in order to improve the sealing between the seal body and the plunger, the fill ratio of the seal to the sealing cavity is set as high as possible in the prior art, and even the volume of the seal is equal to or slightly larger than the volume of the sealing cavity. Then the high sealing is achieved by the plunger pressing the volume of the seal. The design in the prior art is also in line with the general understanding in the field of mechanics.

However, after analyzing a large amount of experimental data, the inventors of the present application found that the environment of the seal between the plunger and the pump body of the atomizer is different from the usual use environment of the seal, which causes the deterioration of the sealing of atomizer in the prior art after a long time or repeated use. In particular, the seal is usually fixed relatively stationary in its environment, such as a sealing plug disposed at the opening of a liquid container. In this environment, the seal is subject to static friction that generally does not cause wear on the seal. Unlike this usual use environment, the seal in the liquid pump of atomizer is fixed to the housing, and the plunger may move (reciprocate) relative to the housing and the seal on the housing. In addition, a large pressure, such as 20MPa to 100MPa, will be generated inside the atomizer during the process of spraying the jet from the atomizer, which will directly act on the seal in the pump. Therefore, the seal of the atomizer will be subjected to dynamic friction and high working pressure, which will cause significant wear on the surfaces of the seals, and it is difficult to maintain the ideal sealing on the surfaces of the worn seals. In addition, the particles generated by the wear of the seal may flow into the liquid and cause contamination of the liquid.

To solve the above problems, the inventor of the present application appropriately reduces the fill ratio of the seal to the sealing cavity, so that a certain gap is left in the sealing cavity to accommodate the deformed seal under pressure. Unexpectedly, the reserved gap in the sealing cavity effectively reduces the wear of the seal, and the entire liquid pump can still have good sealing.

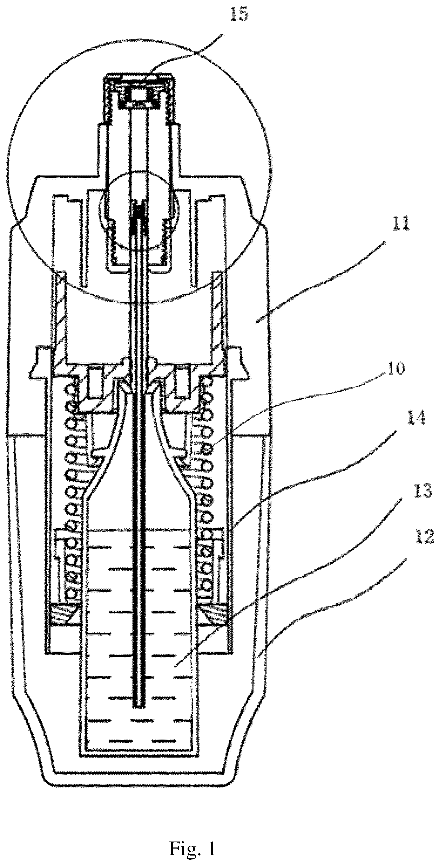

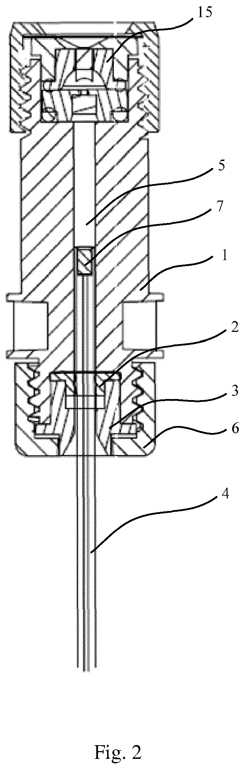

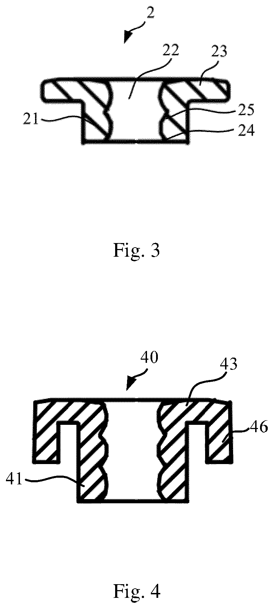

An atomizer according to an embodiment of the present application will be described with Figs.1 to 3. Fig. 1 is a schematic diagram of an atomizer according to a specific embodiment of the present application, Fig. 2 is a partial enlarged view of a liquid pump of the atomizer of Fig. 1, and Fig. 3 is a schematic diagram of a seal of the liquid pump of the atomizer of Fig. 1.

Specifically, the liquid pump of the atomizer includes a pump housing 1, a seal 2, a pressing member 3 and a plunger 4. The pump housing 1 includes an internal chamber 5 that extends in its axial direction, and a mounting seat at a first end of the pump housing 1 and in communication with the internal chamber 5, which is used for accommodating the seal 2 and the pressing member 3. The pump housing 1 also has a second end opposite to the first end, and a nozzle 15 is mounted on the second end. The seal 2 includes a sealing sleeve 21, and a first bore 22 in the sealing sleeve 21 and coaxial with the internal chamber 5 of the pump housing 1. The seal 2 also includes a flange 23 protruding outwardly from the sealing sleeve 21, and the flange 23 rests against a mounting surface of the mounting seat. Referring to the embodiment shown in Fig. 3, the flange 23 of the seal 2 may be at the end of the sealing sleeve 21, and when the seal 2 is mounted in the mounting seat, the flange 23 rests against the bottom surface of the mounting seat. In some other embodiments, the flange 23 of the seal 2 may also be in the middle of the sealing sleeve 21, or at the other end of the sealing sleeve 21 opposite to the position shown in Fig. 3. It can be seen that, an extension direction of the flange 23 is substantially perpendicular to a flow direction of the liquid in the liquid pump (generally flowing in the internal chamber 5). Therefore, the flange 23 can fix the seal 2 on the mounting seat of the pump housing 1 more tightly, thereby avoiding the movement of the seal 2 due to liquid flow or other forces, which may affect the sealing.

In some embodiments, the pressing member 3 may be partially mounted in the mounting seat and rest against the flange 23 of the seal 2 to press and keep it in the mounting seat. The pressing member 3 includes a second bore coaxial with the internal chamber 5 of the pump housing 1 and the first bore 22 of the seal 2. It will be appreciated that a sealing cavity to accommodate the seal 2 is defined by the pump housing 1, the pressing member 3, and the plunger 4 together.

The first end of the plunger 4 can go through the first bore 22 of the seal 2 and the second bore of the pressing member 3 into the internal chamber 5 of the pump housing 1, and can reciprocate in the internal chamber 5 to pump fluid in and out of the internal chamber 5 of the pump housing 1.

It can be understood that, in some embodiments, the seal may not include a flange, but be fixed on the pump housing by other structures and means to limit the movement of the seal in the sealing cavity, especially a movement in the liquid flow direction. For example, the seal may have a substantially same height as the sealing cavity, and an inner side wall of the first bore in the seal may include one or more rings of annular recesses (similar to the recesses between annular corrugated protrusions shown in Fig. 3). In this way, when the seal is under pressure, the annular recess on the inner side wall of the first bore in the seal can release the stress generated by the pressure to effectively avoid friction loss of the seal.

As shown in Fig. 2, the atomizer also includes an external retainer 6. The inner side wall of the external retainer 6 includes an internal thread, and the outer side wall of the mounting seat of the pump housing 1 includes an external thread. The external retainer 6 can fix the pressing member 3 in the mounting seat through a thread connection between the external thread and the internal thread. In other embodiments, the atomizer may not include an external retainer. However, the inner side wall of the mounting seat includes an internal thread, the outer side wall of pressing member includes an external thread, and the mounting seat and the pressing member press the seal through thread connection. In other embodiments, the pressing member includes a brim structure that covers the outer side wall of the mounting seat. The brim structure includes an internal thread, the outer side wall of the mounting seat includes an external thread, and the mounting seat and the pressing member can be connected by the threads to press the seal.

Referring to Fig. 1 and Fig. 2, the atomizer further includes a reservoir 13, a check valve 7 and a nozzle 15, and the reservoir 13 is used for storing a fluid medicine to be atomized for the purpose of treatment. The second end of the plunger 4, which is opposite to the first end extending into the internal chamber 5, extends into the reservoir 13 and contacts with the fluid medicine. The plunger includes a fluid channel therein and the fluid in the reservoir 13 can flow from the second end of the plunger 4 to the first end through the fluid channel, thereby going into the internal chamber 5 of the pump housing 1. In some embodiments, the reservoir 13 is disposable along with the atomizer. In other embodiments, the reservoir 13 is replaceable.

The check valve 7 is disposed on the first end of the plunger 4. When the plunger 4 moves in a direction away from the internal chamber 5, a negative pressure is formed in the internal chamber 5, the check valve 7 is open, and the fluid of the reservoir 13 flows into the internal chamber 5 through the fluid channel in the plunger 4 and the open check valve 7. When the plunger 4 moves in a direction towards the internal chamber 5, the check valve 7 is closed to prevent the fluid in the internal chamber 5 from flowing back to the fluid channel. The check valve 7 may be installed or integrated with the plunger 4, and the specific structure of the check valve 7 may refer to the prior art, which will not be elaborated herein.

The nozzle 15 is disposed at the second end of the pump housing 1 opposite to the aforementioned first end with the mounting seat, and the nozzle 15 includes one or more micro-channels connecting with the internal chamber 5. When the plunger 4 moves in a direction toward the internal chamber 5, the fluid in the internal chamber 5 is squeezed out of the atomizer through the micro-channels of the nozzle 15 to form a jet or aerosol.

Still referring to Fig. 1, the atomizer further includes an upper housing 11 and a lower housing 12, and the reservoir 13 is disposed in the lower housing 12. A spring 10 and a spring limiting housing 14 are also disposed in the lower housing 12. The upper housing 11 includes an upper rotating member, the lower housing 12 includes a lower rotating member, and the inner walls of the upper rotating member and the lower rotating member respectively include sliding surfaces that fit with each other. When an external force is applied to rotate the lower rotating member relative to the upper rotating member in a first direction, the spring 10 is compressed, and at the same time, the plunger 4 moves in a direction away from the internal chamber 5 simultaneously, the check valve 7 is open, and the fluid in the reservoir 13 flows into the internal chamber 5 through the fluid channel in the plunger 4. When the external force is removed, the spring 10 quickly rebounds and is reset, the lower rotating member rotates relative to the upper rotating member in a second direction opposite to the first direction, the plunger 4 moves in a direction towards the internal chamber 5, the check valve 7 is closed, and the fluid in the internal chamber 5 is squeezed out of the atomizer through the micro-channels in the nozzle 15 to form a jet or aerosol. In this embodiment, the atomizer is powered by the pre-compressed elastic force of the spring 10 to complete the atomization of the fluid medicine. In some other embodiments, a motor driven by battery may be used to generate power to complete the atomization of the fluid medicine, which will not be elaborated herein.

Still referring to Fig. 3, it shows a schematic diagram of the seal 2 of the atomizer. As mentioned above, the seal 2 includes a flange 23 protruding outwards from the sealing sleeve 21 in the circumferential direction, and the pressing member 3, when mounted in the mounting seat, rests against the flange 23 of the seal 2 to press and keep it in the mounting seat. The flange 23 can reduce or prevent the displacement of the pressing member 2 caused by the frictional force generated by the reciprocating motion of the plunger 4. In addition, as shown in Fig. 3, a chamfer 24 is formed at an end of the side wall of the first bore 22 of the seal 2 that is away from the mounting surface of the mounting seat. The chamfer 24 may be a guide for the assembly of the plunger 4 into the first bore 22 and the reciprocating motion of the plunger 4 in the first bore 22. Further, as shown in Fig. 3, the side wall of the first bore 22 of the seal 2 includes a plurality of annular corrugated protrusions 25 that surround the plunger 4 flowing through the first bore 22. The annular corrugated protrusion 25 reduces the contact area between the plunger 4 and the seal 2 and increases the pressure therebetween, which is beneficial to improve the sealing and reduce wear. In addition, the plurality of annular corrugated protrusions 25 provide the seal 2 with a multi-layer sealing function. When one of the annular corrugated protrusions 25 is worn out or fails, the other annular corrugated protrusions 25 can be used for sealing.

The material of the seal 2 may include at least one of the following: rubber, silicone, thermoplastic elastomer (TPE) or thermoplastic polyurethane elastomer (TPU), or other sealing materials with suitable elasticity. It should be noted that the material of the seal 2 should not contaminate the medicament and not be harmful to the human being. In addition, although only a cross-sectional view of the seal 2 is shown in FIG. 3, it should be understood that the seal 2 is generally a closed annular shape, for example, the seal 2 may be a circular ring, an elliptical ring or other suitable shapes of closed rings on the section perpendicular to the direction of Fig. 2.

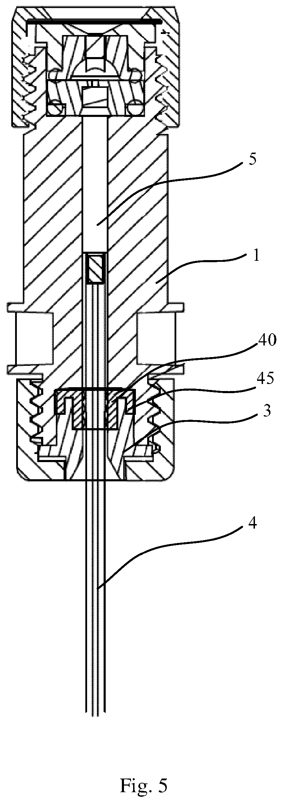

Referring to Figs. 4 and 5, Fig. 4 shows a schematic diagram of a seal 40 according to another embodiment of the present application, and Fig. 5 is a schematic diagram of the seal 40 of Fig. 4 after being assembled into the mounting seat of the pump housing 1 of the atomizer. Compared with the seal 2 of Fig. 3, the flange 43 of the seal 40 includes a lug 46 that is disposed on an end of the flange 43 away from the sealing sleeve 41 and extends in the axial direction of the sealing sleeve 41. Correspondingly, the sealing cavity jointly defined by the pump housing 1, the pressing member 3 and the plunger 4 for accommodating the seal 40 includes a groove matched with the lug 46 to accommodate the lug 46. It can be seen that the connection between the lug 46 on the seal 40 and the groove of the sealing cavity can further limit the movement of the seal 40 relative to the sealing cavity. In the example of Fig. 4 and Fig. 5, the lug is disposed on the outer edge of the flange and extends towards the first end of the pump housing, but in other embodiments, the lug may be disposed in the middle area of the flange or extend toward the second end, or both ends of the pump housing.

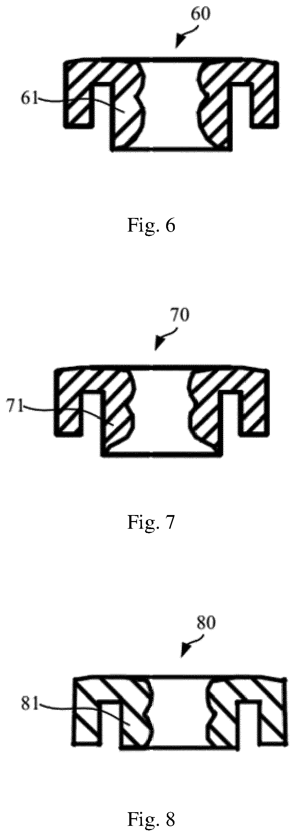

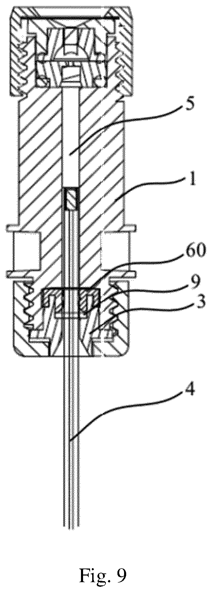

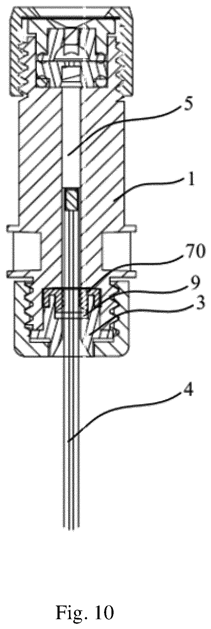

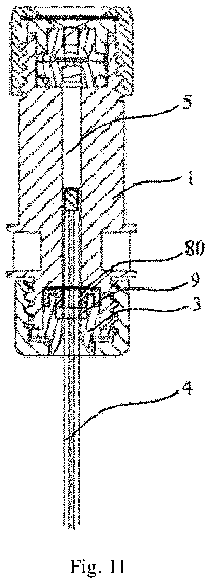

Referring to Figs. 6 to 8, they show schematic diagrams of seals 60, 70 and 80 according to other embodiments of the present application, respectively. It can be seen that the heights of sealing sleeves 61, 71 and 81 of the seals 60, 70 and 80 are different. Referring to Figs. 9-11, when the seals 60, 70 and 80 are assembled into the atomizers, a portion of the sealing cavity away from the flange of each of the seals 60, 70 and 80 in the direction of movement of the plungers 4 includes a gap 9 that is not filled by the respective seals 60, 70, 80. In some embodiments, the height of the gap 9 ranges 5% to 60% of the height of the sealing cavity in the direction of movement of the plunger 4. For example, the heights of gaps 9 of Figs. 9-11 respectively are 10%, 20% and 30% of the heights of the sealing cavities in the direction of movement of the plungers 4. In the examples shown in Figs. 9 to 11, the gaps 9 occupy the entire area of the sealing cavities except for the plungers. In some other examples, the gap may also only partially occupy the area of the sealing cavity except for the plunger. For example, it can be seen from a cross-sectional direction of the plunger 4, an area relatively far from the plunger is another gap, and the area relatively close to the plunger is occupied by the seal. The gap 9 and/or other unfilled areas within the seal cavity may allow seals 60, 70 and 80 to fill less than 90% of the sealing cavities. Alternatively, when the inner surfaces of the seals 60, 70 and 80 have a plurality of corrugated protrusions, the gap 9 together with gaps between the plurality of corrugated protrusions make the fill ratio of the seals 60, 70 and 80 to the sealing cavities less than 90%, such as 50%, 60%, 70%, 80%, 85% or 89%.

Further, the inventor tested the sealing effect of atomizers with different fill ratios and found that if the fill ratio of the sealing cavity is too low, it may result in too much gap left in the sealing cavity, which in turn will reduce the sealing of the atomizer. According to experimental data, the preferred fill ratio of the sealing cavity is 60% to 89%, more preferably 75% to 89%, or most preferably 80% to 89%.

According to the above arrangements, more gaps are reserved in the sealing cavity to provide a movement space for the reciprocating motion of the plunger to squeeze the seal, so that the seal is not easily worn. In addition, as shown in Fig. 9, Fig. 10 and Fig. 11, the gaps 9 in the sealing cavity is in the direction of the movement of the plungers, but the plungers are still in close contact with the seals in the direction perpendicular to the movement of plungers (that is, the cross section of the first bore), thereby reducing wear and ensuring sealing.

For the atomizer with the structure shown in Fig. 1, the operation of the liquid pump pumping out the liquid is driven by the spring 10, and the spring usually has a fixed elastic coefficient. In other words, the driving force and energy provided by the spring 10 during the process of pumping out and atomizing the liquid are basically constant. In the atomizer of the present application, the sealing cavity of the liquid pump is properly filled, which can effectively reduce the wear of the seal, so that the overall sealing effect of the atomizer is generally stable. The good sealing ensures that the dose of the atomizer remains basically unchanged each time, and there is sufficient pressure in the pump body for atomizing the liquid when the liquid is pumped out and atomized. In this way, the particle size of the liquid particles generated by the atomizer is relatively stable, which can meet the actual needs of drug administration. In addition, the reduction of friction loss of the seal can also reduce the entry of particles generated by wear into the medicinal solution, which effectively solves the problem of medicinal solution contamination.

Other modifications to the disclosed embodiments can be understood and implemented by those of ordinary skill in the art from a study of the specification, disclosure and drawings, and the appended claims. In the claims, the word "comprising" does not exclude other elements or steps and the word "a", "an" do not exclude plurals. In a practical application of the present application, one component may perform the functions of several technical features recited in the claims. Any reference signs in the claims shall not be construed as limiting the scope.

## Claims

1. An atomizer, comprising:
a pump housing (1) comprising an internal chamber (5) extending in an axial direction of the pump housing (1) and a mounting seat disposed at a first end of the pump housing (1) and being in communication with the internal chamber (5);
a seal (2; 40; 60; 70; 80) disposed in the mounting seat, the seal (2; 40; 60; 70; 80) comprising a sealing sleeve (21; 41; 61; 71; 81) and a first bore (22) in the sealing sleeve (21; 41; 61; 71; 81) and coaxial with the internal chamber (5), and at least a part of the seal (2; 40; 60; 70; 80) resting against a mounting surface of the mounting seat;
a pressing member (3) at least partially disposed within the mounting seat and resting against the seal (2; 40; 60; 70; 80) to press and keep the seal (2; 40; 60; 70; 80) in the mounting seat, the pressing member (3) comprising a second bore coaxial with the internal chamber (5); and
a plunger (4), a first end of the plunger (4) being disposed within the internal chamber (5) of the pump housing (1) through the second bore of the pressing member (3) and the first bore (22) of the seal (2; 40; 60; 70; 80), and the first end of the plunger (4) being capable of reciprocating in the internal chamber (5) to pump fluid in or out of the internal chamber (5);
wherein the pump housing (1), the pressing member (3) and the plunger (4) together define a sealing cavity to accommodate the seal (2; 40; 60; 70; 80), and when the seal (2; 40; 60; 70; 80) is in the sealing cavity, a fill ratio of the seal (2; 40; 60; 70; 80) to the sealing cavity is less than 90%
**characterized in that**
the seal (2; 40; 60; 70; 80) comprises a flange (23; 43) protruding outward from the sealing sleeve (21; 41; 61; 71; 81), and the flange (23; 43) rests against the mounting surface of the mounting seat; and
a portion of the sealing cavity away from the flange (23; 43) of the seal (2; 40; 60; 70; 80) in a direction of movement of the plunger (4) comprises a gap (9) that is not filled by the seal (2; 40; 60; 70; 80).

2. The atomizer of claim 1, wherein a height of the gap (9) is 5% to 60% of a height of the sealing cavity in the direction of movement of the plunger (4).

3. The atomizer of claim 1, wherein a lug (46) is disposed on the flange (43) of the seal (40; 60; 70; 80), the lug (46) extends in an axial direction of the sealing sleeve (41; 61; 71; 81), the sealing cavity comprises a groove that matches with the lug (46) to accommodate the lug (46), and a movement of the seal (40; 60; 70; 80) relative to the sealing cavity is further limited by the lug (46) and the groove.

4. The atomizer of any one of claims 1 to 3, wherein a side wall of the first bore (22) of the seal (2; 40; 60; 70; 80) comprises a plurality of annular corrugated protrusions (25) surrounding the plunger (4).

5. The atomizer of claim 1, wherein a chamfer (24) is formed at an end of a side wall of the first bore (22) of the seal (2; 40; 60; 70; 80) that is away from the mounting surface.

6. The atomizer of claim 1, wherein the seal (2; 40; 60; 70; 80) comprises at least one material selected from the group consisting of rubber, silicone, or thermoplastic elastomer.

7. The atomizer of claim 1, further comprising:
an external retainer (6), wherein the external retainer (6) comprises an internal thread at its an inner wall, the mounting seat of the pump housing (1) comprises an external thread at its outer wall, and the external retainer (6) is configured to fix the pressing member (3) in the mounting seat through a thread connection between the internal thread and the external thread.

8. The atomizer of claim 1, wherein the fill ratio of the seal (2; 40; 60; 70; 80) to the sealing cavity is 60% to 89%.

9. The atomizer of claim 8, wherein the fill ratio of the seal (2; 40; 60; 70; 80) to the sealing cavity is 75% to 89%.

10. The atomizer of claim 9, wherein the fill ratio of the seal (2; 40; 60; 70; 80) to the sealing cavity is 80% to 89%.

11. The atomizer of claim 1, further comprising:
a reservoir (13), wherein the plunger (4) comprises a fluid channel, and the fluid in the reservoir (13) is capable of flowing through the fluid channel into the internal chamber (5) of the pump housing (1).

12. The atomizer of claim 11, wherein a check valve (7) is disposed on the first end of the plunger (4); the check valve (7) is configured that, when the plunger (4) moves in a direction away from the internal chamber (5), the check valve (7) is open and the fluid in the reservoir (13) flows into the internal chamber (5) through the fluid channel and the check valve (7), and when the plunger (4) moves in a direction toward the internal chamber (5), the check valve (7) is closed to block the fluid from flowing back to the fluid channel.

13. The atomizer of claim 12, further comprising:
a nozzle (15) disposed at a second end of the pump housing (1) opposite to the first end, the nozzle (15) comprising one or more micro-channels in communication with the internal chamber (5);
wherein when the plunger (4) moves in the direction toward the internal chamber (5), the fluid in the internal chamber (5) is squeezed out of the atomizer through the micro-channels of the nozzle (15) to form a jet or aerosol.

## Patentansprüche

1. Zerstäuber, umfassend:
ein Pumpengehäuse (1) mit einer Innenkammer (5), die sich in axialer Richtung des Pumpengehäuses (1) erstreckt, und einem Befestigungssitz, der an einem ersten Ende des Pumpengehäuses (1) angeordnet ist und mit der Innenkammer (5) in Verbindung steht;
eine Dichtung (2; 40; 60; 70; 80), die in dem Befestigungssitz angeordnet ist, wobei die Dichtung (2; 40; 60; 70; 80) eine Dichtungshülse (21; 41; 61; 71; 81) und eine erste Bohrung (22) in der Dichtungshülse (21; 41; 61; 71; 81), die koaxial zur Innenkammer (5) verläuft, wobei zumindest ein Teil der Dichtung (2; 40; 60; 70; 80) an einer Montagefläche des Montagesitzes anliegt;
ein Drückelement (3), das zumindest teilweise innerhalb des Befestigungssitzes angeordnet ist und an der Dichtung (2; 40; 60; 70; 80) anliegt, um die Dichtung (2; 40; 60; 70; 80) in den Befestigungssitz zu drücken und dort zu halten, wobei das Drückelement (3) eine zweite Bohrung aufweist, die koaxial zur Innenkammer (5) ist; und
einen Kolben (4), wobei ein erstes Ende des Kolbens (4) durch die zweite Bohrung des Drückelements (3) und die erste Bohrung (22) der Dichtung (2; 40; 60; 70; 80) in der Innenkammer (5) des Pumpengehäuses (1) angeordnet ist, und wobei das erste Ende des Kolbens (4) in der Innenkammer (5) hin- und herbewegbar ist, um Fluid in die Innenkammer (5) hinein oder aus dieser heraus zu pumpen;
wobei das Pumpengehäuse (1), das Drückelement (3) und der Kolben (4) gemeinsam einen Dichtungshohlraum zur Aufnahme der Dichtung (2; 40; 60; 70; 80) bilden, und dann, wenn sich die Dichtung (2; 40; 60; 70; 80) in dem Dichtungshohlraum befindet, ein Füllverhältnis der Dichtung (2; 40; 60; 70; 80) zum Dichtungshohlraum weniger als 90 % beträgt,
**dadurch gekennzeichnet, dass**
die Dichtung (2; 40; 60; 70; 80) einen Flansch (23; 43) aufweist, der von der Dichtungshülse (21; 41; 61; 71; 81) nach außen vorsteht, und der Flansch (23; 43) an der Montagefläche des Montagesitzes anliegt; und
ein Abschnitt des Dichtungshohlraums, der in Bewegungsrichtung des Kolbens (4) vom Flansch (23; 43) der Dichtung (2; 40; 60; 70; 80) entfernt ist, einen Spalt (9) aufweist, der nicht von der Dichtung (2; 40; 60; 70; 80) ausgefüllt wird.

2. Zerstäuber nach Anspruch 1, wobei die Höhe des Spalts (9) 5 % bis 60 % der Höhe des Dichtungshohlraums in Bewegungsrichtung des Kolbens (4) beträgt.

3. Zerstäuber nach Anspruch 1, wobei an dem Flansch (43) der Dichtung (40; 60; 70; 80) eine Nase (46) angeordnet ist, wobei sich die Nase (46) in axialer Richtung der Dichtungshülse (41; 61; 71; 81) erstreckt, der Dichtungshohlraum eine Nut umfasst, die mit der Nase (46) zusammenpasst, um die Nase (46) aufzunehmen, und eine Bewegung der Dichtung (40; 60; 70; 80) relativ zum Dichtungshohlraum durch die Nase (46) und die Nut weiter begrenzt wird.

4. Zerstäuber nach einem der Ansprüche 1 bis 3, wobei eine Seitenwand der ersten Bohrung (22) der Dichtung (2; 40; 60; 70; 80) eine Vielzahl von ringförmigen, gewellten Vorsprüngen (25) aufweist, die den Kolben (4) umgeben.

5. Zerstäuber nach Anspruch 1, wobei an einem Ende einer Seitenwand der ersten Bohrung (22) der Dichtung (2; 40; 60; 70; 80), das von der Befestigungsfläche entfernt ist, eine Fase (24) ausgebildet ist.

6. Zerstäuber nach Anspruch 1, wobei die Dichtung (2; 40; 60; 70; 80) mindestens ein Material umfasst, das aus der Gruppe ausgewählt ist, die aus Gummi, Silikon oder thermoplastischem Elastomer besteht.

7. Zerstäuber nach Anspruch 1, der ferner umfasst:
einen äußeren Halter (6), wobei der äußere Halter (6) an seiner Innenwand ein Innengewinde aufweist, der Befestigungssitz des Pumpengehäuses (1) an seiner Außenwand ein Außengewinde aufweist und der äußere Halter (6) so ausgebildet ist, dass er das Drückelement (3) durch eine Gewindeverbindung zwischen dem Innengewinde und dem Außengewinde im Befestigungssitz fixiert.

8. Zerstäuber nach Anspruch 1, wobei das Füllverhältnis der Dichtung (2; 40; 60; 70; 80) zum Dichtungshohlraum 60 % bis 89 % beträgt.

9. Zerstäuber nach Anspruch 8, wobei das Füllverhältnis der Dichtung (2; 40; 60; 70; 80) zum Dichtungshohlraum 75 % bis 89 % beträgt.

10. Zerstäuber nach Anspruch 9, wobei das Füllverhältnis der Dichtung (2; 40; 60; 70; 80) zum Dichtungshohlraum 80 % bis 89 % beträgt.

11. Zerstäuber nach Anspruch 1, der ferner Folgendes umfasst:
einen Vorratsbehälter (13), wobei der Kolben (4) einen Fluidkanal aufweist und das Fluid im Vorratsbehälter (13) durch den Fluidkanal in die Innenkammer (5) des Pumpengehäuses (1) fließen kann.

12. Zerstäuber nach Anspruch 11, wobei am ersten Ende des Kolbens (4) ein Rückschlagventil (7) angeordnet ist; das Rückschlagventil (7) ist so ausgebildet, dass dann, wenn sich der Kolben (4) in einer Richtung weg von der Innenkammer (5) bewegt, das Rückschlagventil (7) geöffnet ist und die Flüssigkeit im Vorratsbehälter (13) durch den Flüssigkeitskanal und das Rückschlagventil (7) in die Innenkammer (5) fließt, und dann, wenn sich der Kolben (4) in Richtung der Innenkammer (5) bewegt, das Rückschlagventil (7) geschlossen ist, um ein Zurückfließen der Flüssigkeit in den Flüssigkeitskanal zu verhindern.

13. Zerstäuber nach Anspruch 12, der ferner Folgendes umfasst:
eine Düse (15), die an einem dem ersten Ende gegenüberliegenden zweiten Ende des Pumpengehäuses (1) angeordnet ist, wobei die Düse (15) einen oder mehrere Mikrokanäle umfasst, die mit der Innenkammer (5) in Verbindung stehen;
wobei dann, wenn sich der Kolben (4) in Richtung der Innenkammer (5) bewegt, das Fluid in der Innenkammer (5) durch die Mikrokanäle der Düse (15) aus dem Zerstäuber herausgedrückt wird, um einen Strahl oder ein Aerosol zu bilden.

## Revendications

1. Atomiseur, comprenant :
un boîtier de pompe (1) comprenant une chambre interne (5) s'étendant dans une direction axiale du boîtier de pompe (1) et un siège de montage disposé à une première extrémité du boîtier de pompe (1) et communiquant avec la chambre interne (5) ;
un joint d'étanchéité (2 ; 40 ; 60 ; 70 ; 80) disposé dans le siège de montage, le joint d'étanchéité (2 ; 40 ; 60 ; 70 ; 80) comprenant un manchon d'étanchéité (21 ; 41 ; 61 ; 71 ; 81) et un premier alésage (22) dans le manchon d'étanchéité (21 ; 41 ; 61 ; 71 ; 81) et coaxial à la chambre interne (5), et au moins une partie du joint d'étanchéité (2 ; 40 ; 60 ; 70 ; 80) reposant contre une surface de montage du siège de montage ;
un élément de pression (3) disposé au moins partiellement à l'intérieur du siège de montage et reposant contre le joint d'étanchéité (2 ; 40 ; 60 ; 70 ; 80) pour presser et maintenir le joint d'étanchéité (2 ; 40 ; 60 ; 70 ; 80) dans le siège de montage, l'élément de pression (3) comprenant un deuxième alésage coaxial à la chambre interne (5) ; et
un piston (4), une première extrémité du piston (4) étant disposée à l'intérieur de la chambre interne (5) du boîtier de pompe (1) à travers le deuxième alésage de l'élément de pression (3) et le premier alésage (22) du joint d'étanchéité (2 ; 40 ; 60 ; 70 ; 80), et la première extrémité du piston (4) étant capable d'effectuer un mouvement de va-et-vient dans la chambre interne (5) pour pomper du fluide vers ou hors de la chambre interne (5) ;
dans lequel le boîtier de pompe (1), l'élément de pression (3) et le piston (4) définissent ensemble une cavité d'étanchéité destinée à recevoir le joint d'étanchéité (2 ; 40 ; 60 ; 70 ; 80), et lorsque le joint d'étanchéité (2 ; 40 ; 60 ; 70 ; 80) se trouve dans la cavité d'étanchéité, le taux de remplissage du joint d'étanchéité (2 ; 40 ; 60 ; 70 ; 80) par rapport à la cavité d'étanchéité est inférieur à 90 %
**caractérisé en ce que**
le joint d'étanchéité (2 ; 40 ; 60 ; 70 ; 80) comprend une bride (23 ; 43) faisant saillie vers l'extérieur à partir du manchon d'étanchéité (21 ; 41 ; 61 ; 71 ; 81), et la bride (23 ; 43) repose contre la surface de montage du siège de montage ; et une partie de la cavité d'étanchéité éloignée de la bride (23 ; 43) du joint d'étanchéité (2 ; 40 ; 60 ; 70 ; 80) dans une direction de déplacement du piston (4) comprend un espace (9) qui n'est pas comblé par le joint d'étanchéité (2 ; 40 ; 60 ; 70 ; 80).

2. L'atomiseur selon la revendication 1, dans lequel la hauteur de l'espace (9) est comprise entre 5 % et 60 % de la hauteur de la cavité d'étanchéité dans la direction de déplacement du piston (4).

3. L'atomiseur selon la revendication 1, dans lequel une patte (46) est disposée sur la bride (43) du joint d'étanchéité (40 ; 60 ; 70 ; 80), la patte (46) s'étendant dans une direction axiale du manchon d'étanchéité (41 ; 61 ; 71 ; 81), la cavité d'étanchéité comprend une rainure qui s'adapte à la patte (46) pour recevoir celle-ci, et le mouvement du joint d'étanchéité (40 ; 60 ; 70 ; 80) par rapport à la cavité d'étanchéité est en outre limité par la patte (46) et la rainure.

4. L'atomiseur selon l'une quelconque des revendications 1 à 3, dans lequel une paroi latérale du premier alésage (22) du joint d'étanchéité (2 ; 40 ; 60 ; 70 ; 80) comprend une pluralité de saillies annulaires ondulées (25) entourant le piston (4).

5. L'atomiseur selon la revendication 1, dans lequel un chanfrein (24) est formé à une extrémité d'une paroi latérale du premier alésage (22) du joint d'étanchéité (2 ; 40 ; 60 ; 70 ; 80) qui est éloignée de la surface de montage.

6. L'atomiseur selon la revendication 1, dans lequel le joint (2 ; 40 ; 60 ; 70 ; 80) comprend au moins un matériau choisi parmi le groupe constitué du caoutchouc, du silicone ou d'un élastomère thermoplastique.

7. L'atomiseur selon la revendication 1, comprenant en outre :
un dispositif de retenue externe (6), dans lequel le dispositif de retenue externe (6) comprend un filetage interne sur sa paroi interne, le siège de montage du boîtier de pompe (1) comprend un filetage externe sur sa paroi externe, et le dispositif de retenue externe (6) est configuré pour fixer l'élément de pression (3) dans le siège de montage par l'intermédiaire d'une connexion filetée entre le filetage interne et le filetage externe.

8. L'atomiseur selon la revendication 1, dans lequel le taux de remplissage du joint (2 ; 40 ; 60 ; 70 ; 80) par rapport à la cavité d'étanchéité est compris entre 60 % et 89 %.

9. L'atomiseur selon la revendication 8, dans lequel le taux de remplissage du joint (2 ; 40 ; 60 ; 70 ; 80) par rapport à la cavité d'étanchéité est compris entre 75 % et 89 %.

10. L'atomiseur selon la revendication 9, dans lequel le taux de remplissage du joint (2 ; 40 ; 60 ; 70 ; 80) par rapport à la cavité d'étanchéité est compris entre 80 % et 89 %.

11. L'atomiseur selon la revendication 1, comprenant en outre :
un réservoir (13), dans lequel le piston (4) comprend un canal de fluide, et le fluide contenu dans le réservoir (13) est capable de s'écouler à travers le canal de fluide dans la chambre interne (5) du boîtier de pompe (1).

12. L'atomiseur selon la revendication 11, dans lequel un clapet anti-retour (7) est disposé sur la première extrémité du piston (4) ; le clapet anti-retour (7) est configuré de telle sorte que, lorsque le piston (4) se déplace dans une direction s'éloignant de la chambre interne (5), le clapet anti-retour (7) est ouvert et le fluide contenu dans le réservoir (13) s'écoule dans la chambre interne (5) à travers le canal de fluide et le clapet anti-retour (7), et lorsque le piston (4) se déplace dans une direction vers la chambre interne (5), le clapet anti-retour (7) est fermé pour empêcher le fluide de refluer vers le canal de fluide.

13. L'atomiseur selon la revendication 12, comprenant en outre :
une buse (15) disposée à une deuxième extrémité du boîtier de pompe (1) opposée à la première extrémité, la buse (15) comprenant un ou plusieurs micro-canaux en communication avec la chambre interne (5) ;
dans lequel, lorsque le piston (4) se déplace en direction de la chambre interne (5), le fluide présent dans la chambre interne (5) est expulsé de l'atomiseur par les micro-canaux de la buse (15) pour former un jet ou un aérosol.
